# EUROPEAN PATENT SPECIFICATION

(11) **EP 4 332 888 B1**
(45) Date of publication and mention of the grant of the patent: **31.12.2025**
(21) Application number: 23176642.9
(22) Date of filing: 01.06.2023
(51) Int. Cl.: G06T 7/00, A61B 6/00, A61B 6/03

(54) **LESION DETECTION METHOD AND LESION DETECTION PROGRAM**
LÄSIONSDETEKTIONSVERFAHREN UND LÄSIONSDETEKTIONSPROGRAMM
PROCÉDÉ ET PROGRAMME DE DÉTECTION DE LÉSION

(30) Priority: 29.08.2022 JP 2022135519
(43) Date of publication of application: 06.03.2024
(73) Proprietor: FUJITSU LIMITED, Kawasaki-shi, Kanagawa 211-8588 (JP)
(72) Inventor: BABA, Kozo, Kawasaki-shi, Kanagawa, 211-8588 (JP); TAKEBE, Hiroaki, Kawasaki-shi, Kanagawa, 211-8588 (JP); MIYAZAKI, Nobuhiro, Kawasaki-shi, Kanagawa, 211-8588 (JP)
(74) Representative: Hoffmann Eitle

(56) References cited:
- WO-A1-2022/051344
- US-A1- 2020 303 062
- US-A1- 2021 093 249
- US-A1- 2022 138 956

## Description

### FIELD

The embodiments discussed herein are related to a lesion detection method and a lesion detection program.

### BACKGROUND

Medical images obtained by computed tomography (CT), magnetic resonance imaging (MRI), and the like are widely used for diagnosis of various diseases. A doctor needs to interpret a large number of images in diagnostic imaging using medical images, which places a heavy burden on the doctor. Therefore, there has been a demand for a technique of supporting a diagnostic work of a doctor in some way using a computer.

As an example of such a technique, there is a technique for detecting a lesion region from a medical image, using a trained model created through machine learning. For example, an image processing device has been proposed that executes primary classification processing for acquiring cross-sectional images in a plurality of different cross-sectional directions and specifying a type of a lesion in each pixel for each cross-sectional image using a discriminator that includes a multilayer neural network and evaluates a result of the primary classification processing for the pixel common to each cross-sectional image. Furthermore, it has been proposed to extend use of a known convolutional neural network (CNN) to a 3D image in order to analyze and segmentalize a 2D image, in a composite computing system that combines N different CNNs along N different planes of a 3D image volume.

Japanese National Publication of International Patent Application No. 2022-525198 and Japanese National Publication of International Patent Application No. 2019-500146 are disclosed as related art.
US 2022/138956 A1 (PALMA GIOVANNI JOHN JACQUES [FR] ET AL), 5 May 2022 (2022-05-05), deals with an operation of a liver/lesion detection with ML/DL computer models. The operation includes performing a segmentation of an input volume to identify a mask for the anatomical structure, e.g., a liver mask by means of a first trained ML/DL model. The input volume is also processed via the first trained ML/DL model to generate a first set of lesion detection predictions. The second trained ML/DL model processes the above masked input via two different decoders and then combines the results of two sets of lesion prediction from the two different decoders to generate a lesion prediction output of the second ML/DL model. If necessary, the lesion prediction output of the second ML/DL model may be combined with the first lesion prediction output generated by the first ML/DL model of the ensemble to generate a final lesion prediction output. The ML/DL models may identify all of the contours corresponding to lesions present in slices of the input volume based on the image element partitioning, and perform operations to identify which contours correspond to the same lesion in three dimensions.
US 2020/303062 A1 (TAO PENG [CN]), 24 September 2020 (2020-09-24), deals with obtaining a main closed region of one or more closed core focus regions corresponding to the focus (that is, a lesion region) in a two-dimensional sub-image (frame) cross-section by dividing certain focuses from the organs to which the focuses connect.

### SUMMARY

### [TECHNICAL PROBLEM]

By the way, methods for detecting a lesion region using a trained model includes, for example, a first method for classifying whether or not each pixel is a lesion using a two-dimensional tomographic image as an input and a second method for classifying whether or not each voxel is a lesion using three-dimensional volume data created based on a plurality of tomographic images as an input. Typically, since the second method can accurately capture a three-dimensional shape feature of the lesion region and detect the lesion region, detection accuracy can be improved. However, with the second method, in a case where a slice interval of the plurality of tomographic images that is a creation source of the volume data at the time of inference is larger than that at the time of training, reliability of a shape of the lesion region in volume data for inference is deteriorated. Therefore, there is a problem such that the detection accuracy of the lesion region is easily decreased.

In one aspect, an object of the embodiment is to provide a method for detecting a lesion and a lesion detection program that can reduce an effect of a slice interval on detection accuracy of a lesion region from a plurality of tomographic images.

### [SOLUTION TO PROBLEM]

The present invention is defined by the independent claims. Preferred embodiments are defined by the dependent claims. Further aspects and examples are provided for facilitating the understanding of the invention. According to an example, a lesion detection method for a computer to execute a process includes detecting a first lesion region that indicates a certain lesion from each of a plurality of tomographic images obtained by imaging an inside of a human body, by using a first machine learning model that detects a lesion region from an input image that has image data of a two-dimensional space; detecting a second lesion region that indicates the certain lesion from three-dimensional volume data generated based on the plurality of tomographic images, by using a second machine learning model that detects a lesion region from input volume data that has image data of a three-dimensional space; and detecting a third lesion region that indicates the certain lesion from each of the plurality of tomographic images, based on an overlapping state between the first lesion region and the second lesion region.

### [ADVANTAGEOUS EFFECTS OF INVENTION]

In one aspect, it is possible to reduce an effect of a slice interval on detection accuracy of a lesion region from a plurality of tomographic images.

### BRIEF DESCRIPTION OF DRAWINGS

FIG. 1 is a diagram illustrating a configuration example and a processing example of a lesion detection device according to a first embodiment;
FIG. 2 is a diagram illustrating a configuration example of a diagnosis support system according to a second embodiment;
FIG. 3 is a diagram illustrating a first example of a machine learning model for detecting a lesion region;
FIG. 4 is a diagram illustrating a second example of the machine learning model for detecting the lesion region;
FIG. 5 is a diagram illustrating an example of a method for detecting a lesion using tomographic images of cross sections in a plurality of directions;
FIG. 6 is a diagram illustrating an example of a lesion shape;
FIG. 7 is a diagram for explaining an effect of a slice interval on segmentation accuracy;
FIG. 8 is a diagram illustrating a configuration example of processing functions included in a diagnosis support device;
FIG. 9 is an example of a flowchart illustrating a procedure of segmentation processing using a 2D model and a 3D model;
FIG. 10 is a first diagram illustrating combination determination processing;
FIG. 11 is a second diagram illustrating the combination determination processing;
FIG. 12 is an example (part 1) of a flowchart illustrating a procedure of the combination determination processing;
FIG. 13 is an example (part 2) of the flowchart illustrating the procedure of the combination determination processing;
FIG. 14 is a diagram illustrating a screen display example of a detection result of the lesion region; and
FIG. 15 is an example of a flowchart illustrating a procedure of combination determination processing according to a modification.

### DESCRIPTION OF EMBODIMENTS

Hereinafter, embodiments will be described with reference to the drawings.

### [First Embodiment]

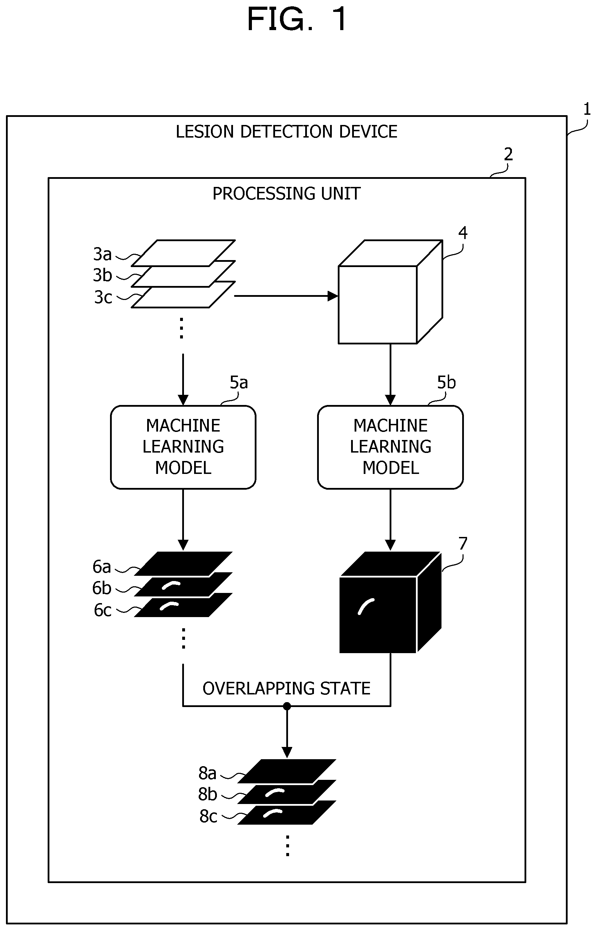

FIG. 1 is a diagram illustrating a configuration example and a processing example of a lesion detection device according to a first embodiment. A lesion detection device 1 illustrated in FIG. 1 is a device that detects a region of a predetermined lesion from a medical image. The lesion detection device 1 includes a processing unit 2. The processing unit 2 is, for example, a processor. In this case, the following processing by the processing unit 2 is realized, for example, by executing a predetermined program by a processor.

The processing unit 2 acquires a plurality of tomographic images 3a, 3b, 3c, ... obtained by imaging inside of a human body, as medical images. The tomographic images 3a, 3b, 3c, ... are, for example, tomographic images obtained by a CT or an MRI. Furthermore, the tomographic images 3a, 3b, 3c, ... are images along a cross-section (slice plane) in the same direction in the inner region of the human body including a predetermined organ and can be obtained by imaging the inner region of the human body while changing a position with respect to a direction perpendicular to the cross section at a predetermined interval (slice interval).

The processing unit 2 detects a first region indicating the lesion described above from each of the tomographic images 3a, 3b, 3c, ..., using a machine learning model 5a that detects the lesion region described above from an input image having image data of a two-dimensional space. With this processing, for example, in each of the tomographic images 3a, 3b, 3c, ..., pixels are classified into lesion regions and other regions. In FIG. 1, as an example of information indicating the first region, detection result images 6a, 6b, 6c, ... in which a pixel value of the lesion region is set as "1" and a pixel value of the other region is set as "0" are output.

Furthermore, the processing unit 2 creates three-dimensional volume data 4 based on the tomographic images 3a, 3b, 3c, .... The processing unit 2 detects a second region indicating the lesion described above from the created volume data 4, using a machine learning model 5b that detects the lesion region described above from input volume data having image data of a three-dimensional space. With this processing, for example, each voxel of the volume data 4 is classified into a lesion region and other regions. In FIG. 1, as an example of information indicating the second region, volume data 7 in which a voxel value of the lesion region is set as "1" and a voxel value of the other region is set as "0" is output.

The processing unit 2 detects a third lesion region indicating the lesion described above from each of the tomographic images 3a, 3b, 3c, ..., based on an overlapping state of the detected first lesion region and the detected second lesion region. For example, the processing unit 2 calculates a lesion region in the three-dimensional space, based on the first lesion region detected from each of the tomographic images 3a, 3b, 3c, ... and recognizes an overlapping state between the calculated lesion region and the second region detected as the region in the three-dimensional space. The processing unit 2 detects the third lesion region from each of the tomographic images 3a, 3b, 3c, ... based on this overlapping state. In FIG. 1, as an example of information indicating the third lesion region, detection result images 8a, 8b, 8c, ... in which a pixel value of the lesion region is set as "1" and a pixel value of the other region is set as "0" are output.

According to the above processing, a final detection result is created using not only the detection result of the lesion region based on the volume data 4 but also the detection result of the lesion region based on the two-dimensional tomographic images 3a, 3b, 3c, .... As a result, it is possible to reduce an effect of a slice interval of the tomographic images 3a, 3b, 3c, ... that are a creation source of the volume data 4 on detection accuracy of the lesion region from the plurality of tomographic images. As a result, it is possible to increase a possibility of increasing the detection accuracy of the lesion region, without depending on the slice interval.

### [Second Embodiment]

Next, a system that can detect a lesion region of a liver from a CT image will be described.

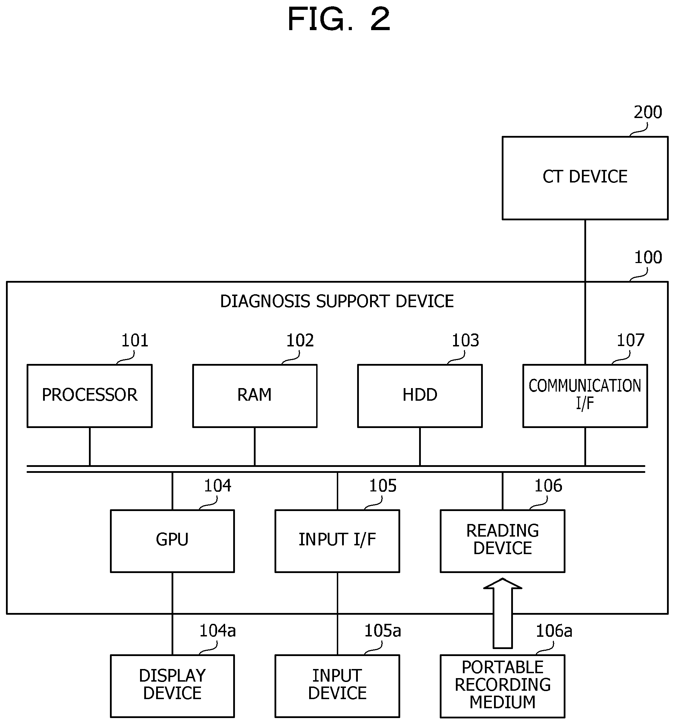

FIG. 2 is a diagram illustrating a configuration example of a diagnosis support system according to a second embodiment. The diagnosis support system illustrated in FIG. 2 is a system that supports an image diagnosis work through CT imaging and includes a diagnosis support device 100 and a CT device 200. Note that the diagnosis support device 100 is an example of the lesion detection device 1 illustrated in FIG. 1.

The CT device 200 captures an X-ray CT image of a human body. In the present embodiment, the CT device 200 captures a predetermined number of tomographic images of axial planes in an abdominal region including the liver, while changing a position (slice position) in a height direction of the human body (direction perpendicular to axial plane) at predetermined intervals.

The diagnosis support device 100 extracts a region of the liver from each tomographic image captured by the CT device 200 and detects a lesion region based on image information of the extracted region. In the present embodiment, it is assumed that intrahepatic bile duct dilatation be detected as the lesion region. For example, the diagnosis support device 100 causes a display device to display information indicating a detection result of a lesion region. As a result, the diagnosis support device 100 supports the image diagnosis work of a user (for example, radiologist).

Hereinafter, a hardware configuration of the diagnosis support device 100 will be described with reference to FIG. 2. The diagnosis support device 100 is implemented, for example, as a computer illustrated in FIG. 2. As illustrated in FIG. 2, the diagnosis support device 100 includes a processor 101, a random access memory (RAM) 102, a hard disk drive (HDD) 103, a graphics processing unit (GPU) 104, an input interface (I/F) 105, a reading device 106, and a communication interface (I/F) 107.

The processor 101 integrally controls the entire diagnosis support device 100. The processor 101 is, for example, a central processing unit (CPU), a micro processing unit (MPU), a digital signal processor (DSP), an application specific integrated circuit (ASIC), or a programmable logic device (PLD). Furthermore, the processor 101 may be a combination of two or more elements of a CPU, an MPU, a DSP, an ASIC, and a PLD. Note that the processor 101 is an example of the processing unit 2 illustrated in FIG. 1.

The RAM 102 is used as a main storage device of the diagnosis support device 100. The RAM 102 temporarily stores at least a part of an operating system (OS) program or an application program to be executed by the processor 101. Furthermore, the RAM 102 stores various types of data needed for processing by the processor 101.

The HDD 103 is used as an auxiliary storage device of the diagnosis support device 100. The HDD 103 stores the OS program, the application program, and various types of data. Note that another type of nonvolatile storage device, such as a solid state drive (SSD), may be used as the auxiliary storage device.

A display device 104a is coupled to the GPU 104. The GPU 104 displays an image on the display device 104a in accordance with an instruction from the processor 101. Examples of the display device 104a include a liquid crystal display, an organic electroluminescence (EL) display, or the like.

An input device 105a is coupled to the input interface 105. The input interface 105 transmits a signal output from the input device 105a to the processor 101. Examples of the input device 105a include a keyboard, a pointing device, or the like. Examples of the pointing device include a mouse, a touch panel, a tablet, a touch pad, a track ball, or the like.

A portable recording medium 106a is attached/detached to/from the reading device 106. The reading device 106 reads data recorded in the portable recording medium 106a and transmits the data to the processor 101. Examples of the portable recording medium 106a include an optical disk, a semiconductor memory, or the like.

The communication interface 107 exchanges data with another device such as the CT device 200, via a network.

The processing functions of the diagnosis support device 100 can be implemented with the hardware configuration described above.

By the way, processing for detecting a lesion region from a medical image can be executed, for example, using a machine learning model.

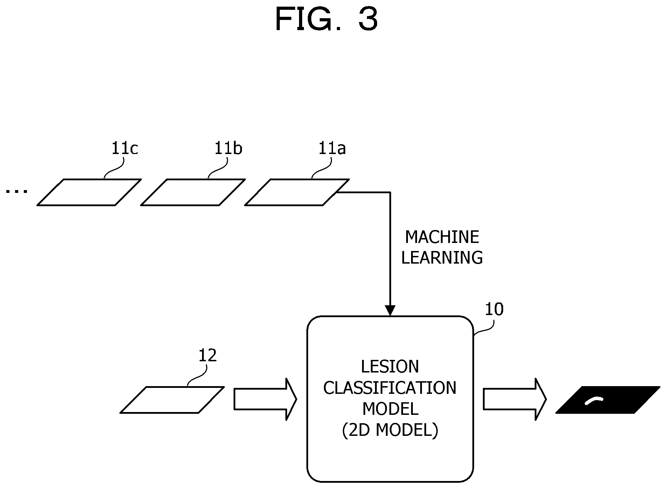

FIG. 3 is a diagram illustrating a first example of a machine learning model for detecting a lesion region. A lesion classification model 10 illustrated in FIG. 3 is a trained model that classifies whether or not a pixel is a specific lesion region for each pixel of a tomographic image 12, upon receiving an input of the tomographic image 12. Here, it is assumed that the tomographic image 12 be a tomographic image of an axial plane.

The lesion classification model 10 is created through machine learning (for example, deep learning) similarly using tomographic images 11a, 11b, 11c, ... of the axial plane as teacher data. A label indicating whether or not each pixel is a lesion region is added to these tomographic images 11a, 11b, 11c, ..., and these labels are used as correct answer data at the time of machine learning.

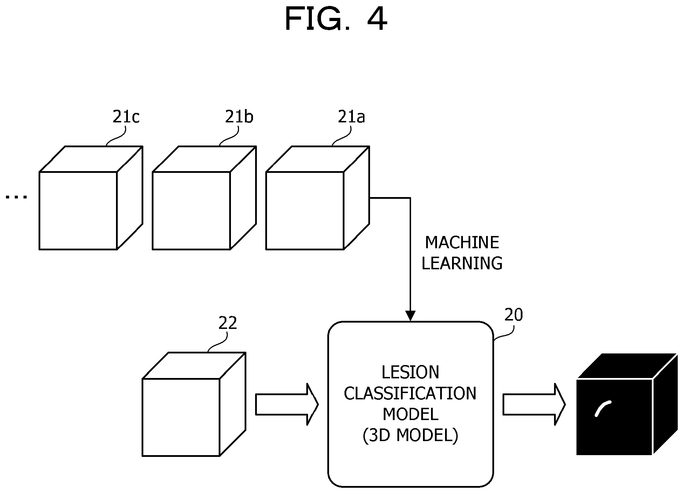

FIG. 4 is a diagram illustrating a second example of the machine learning model for detecting the lesion region. Three-dimensional volume data can be created, based on a plurality of tomographic images (tomographic image set) imaged while changing a slice position by the CT device 200. A lesion classification model 20 illustrated in FIG. 4 is a trained model that classifies whether or not each voxel of volume data 22 is a specific lesion region, upon receiving an input of such volume data 22.

The lesion classification model 20 is created through machine learning (for example, deep learning) using pieces of volume data 21a, 21b, 21c, ... as teacher data. A label indicating whether or not each voxel is a lesion region is added to these pieces of volume data 21a, 21b, 21c, ..., and these labels are used as correct answer data at the time of machine learning.

Hereinafter, the lesion classification model 10 described above that performs segmentation on the tomographic image formed of image data of a two-dimensional space is described as a "2D model 10". On the other hand, the lesion classification model 20 described above that performs segmentation on the volume data formed of image data of a three-dimensional space is described as a "3D model 20".

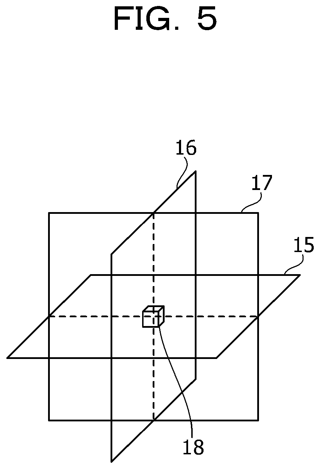

Here, with the segmentation using the 2D model 10, it is possible to detect a lesion with a shape along a surface of the tomographic image with relatively high accuracy. However, there is a problem in that detection accuracy of a lesion with a shape that is not three-dimensionally along the surface of the tomographic image is low. For example, in a case where a tubular lesion is not along the surface of the tomographic image, the lesion is projected as a circular shape or an elliptical shape on the tomographic image. Therefore, it is difficult to distinguish the lesion from a spherical lesion such as a tumor. As an example of a method for solving this problem, the following method illustrated in FIG. 5 is considered.

FIG. 5 is a diagram illustrating an example of a method for detecting a lesion using tomographic images of cross sections in a plurality of directions. In the method illustrated in FIG. 5, a lesion region is detected using each of tomographic images of an axial plane, a sagittal plane, and a coronal plane.

Specifically, a first 2D model created through machine learning using the tomographic image of the axial plane, a second 2D model created through machine learning using the tomographic image of the sagittal plane, and a third 2D model created through machine learning using the tomographic image of the coronal plane are used. Then, segmentation of a tomographic image 15 of the axial plane is performed using the first 2D model, segmentation of a tomographic image 16 of the sagittal plane is performed using the second 2D model, and segmentation of a tomographic image 17 of the coronal plane is performed using the third 2D model. As a result, a classification result of a pixel 18 common to the tomographic images 15 to 17 is calculated from each 2D model, and a final classification result of the pixel 18 is obtained, for example, by a majority vote, based on these classification results.

With such a method, it is possible to increase segmentation accuracy that that in a case where a tomographic image of one cross section is used. However, even if the tomographic images of the cross sections in the plurality of directions are used, segmentation using a 2D model that captures shape features in the two-dimensional space is performed. Therefore, it is not possible to perform highly accurate segmentation depending on the lesion shape in the three-dimensional space.

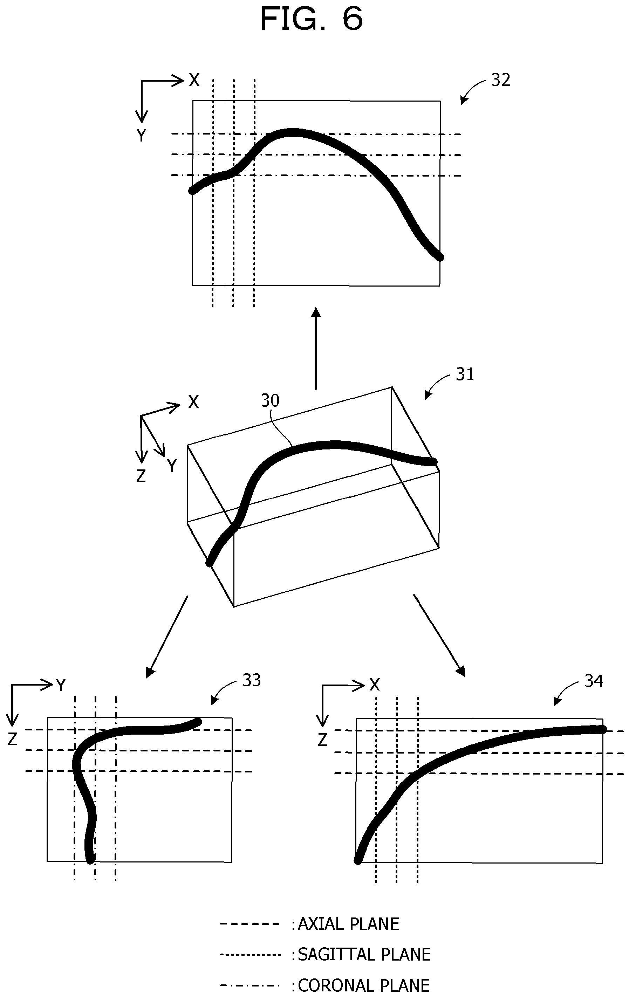

FIG. 6 is a diagram illustrating an example of a lesion shape. In FIG. 6, an image 32 is an image in a case where a lesion region 30 is viewed from the upper side of a human body (viewed in Z-axis direction) and corresponds to the tomographic image of the axial plane. An image 33 is an image in a case where the lesion region 30 is viewed from the left side of the human body (viewed in X-axis direction) and corresponds to the tomographic image of the sagittal plane. An image 34 is an image in a case where the lesion region 30 is viewed from the front side of the human body (viewed in direction opposite to Y-axis direction) and corresponds to the tomographic image of the coronal plane.

As illustrated in a perspective view 31, the shape of this lesion region 30 is a tubular shape having no symmetry with respect to all of the X axis, the Y axis, and the Z axis. In this case, an entire image of the lesion region 30 is not imaged in any one of the tomographic images of the axial plane, the sagittal plane, and the coronal plane. Therefore, even if the method illustrated in FIG. 5 is used, it cannot be said that segmentation for sufficiently capturing the shape of the lesion region 30 in the three-dimensional space is performed, and it is difficult to perform highly accurate segmentation. In other words, it is difficult to perform segmentation while accurately distinguishing a tubular lesion region from a spherical lesion region.

On the other hand, with a method using the 3D model 20 illustrated in FIG. 4, it is possible to perform the segmentation for sufficiently capturing the shape of the lesion region 30 in the three-dimensional space, and it is possible to improve the accuracy of the segmentation. However, in a case where the 3D model 20 is used, a slice interval of a tomographic image set to be a creation source of volume data to be used at the time of training or inference may affect the accuracy of the segmentation.

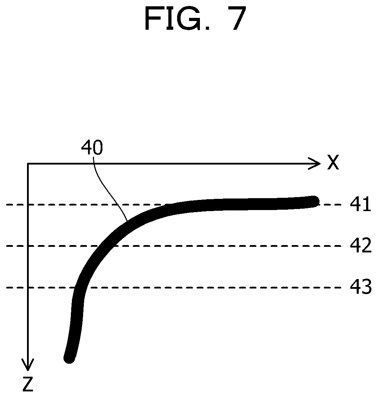

FIG. 7 is a diagram for explaining an effect of a slice interval on the segmentation accuracy.

In a case where the slice interval between the tomographic images is larger than a size of the pixel of each tomographic image included in the tomographic image set, data of a voxel between the tomographic images is calculated through the interpolation operation at the time when the volume data is created. In a case where the tomographic image of the axial plane (X-Y plane) is used as in the present embodiment, an interpolation operation in the vertical direction (Z-axis direction) is performed. Therefore, shape reliability of the lesion region in the Z-axis direction in the volume data is lowered as the slice interval is larger, it is not possible to accurately held a three-dimensional shape of the lesion region in the volume data.

FIG. 7 illustrates a shape of a lesion region 40 in a case of viewed in the Y-axis direction. The shape of the lesion region 40 is asymmetric with respect to all of an X-Y plane, a Y-Z plane, and a Z-X plane. In this case, a case where the lesion region 40 is elongatedly projected on the X-Y plane and a case where the lesion region 40 is elongatedly projected in the Z-axis direction are mixed. The shape of the lesion region indicating the intrahepatic bile duct dilatation is often asymmetric with respect to all of the X-Y plane, the Y-Z plane, and the Z-X plane in this way.

Such a lesion region 40 is imaged like a long linear shape with respect to the X-Y plane on an imaging plane 41, and is imaged like a minute circular shape on imaging planes 42 and 43. Therefore, for example, the shape of the lesion region 40 appearing in the volume data is different between a case where the interpolation operation is performed using the respective tomographic images on the imaging planes 41 and 43 and a case where the interpolation operation is performed using the respective tomographic images on the imaging planes 41 to 43. In other words, in the latter case, the shape of the lesion region 40 appearing in the volume data is closer to an original shape, that the former case.

Therefore, at the time of training the 3D model 20, the 3D model 20 with higher segmentation accuracy can be created as the volume data based on the tomographic image set with shorter slice intervals is used as the teacher data. On the other hand, at the time of inference using the 3D model 20, when the volume data based on the tomographic image set with longer slice intervals than that at the time of training is input to the 3D model 20, there is a possibility that the segmentation accuracy is lowered.

In an actual medical site, from viewpoint of cost reduction, it is often not possible to increase the number of tomographic images captured at a time. Therefore, the slice interval of the tomographic image set used at the time of the inference using the 3D model 20 is often larger than the slice interval of the tomographic image set used at the time of training the 3D model 20. In this case, inference accuracy using the 3D model 20 is lowered.

Therefore, the diagnosis support device 100 according to the present embodiment acquires a final segmentation result, by combining a segmentation result by the 2D model 10 and a segmentation result by the 3D model 20. As a result, it is possible to execute lesion segmentation processing with high accuracy.

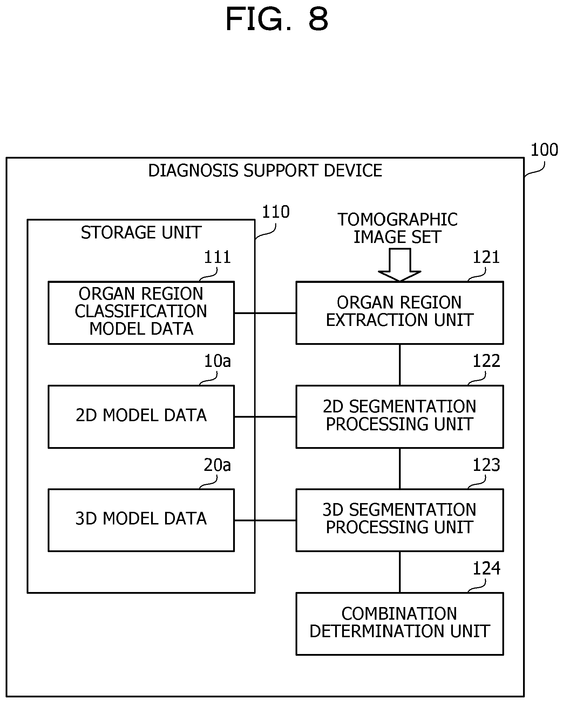

FIG. 8 is a diagram illustrating a configuration example of processing functions included in the diagnosis support device. As illustrated in FIG. 8, the diagnosis support device 100 includes a storage unit 110, an organ region extraction unit 121, a 2D segmentation processing unit 122, a 3D segmentation processing unit 123, and a combination determination unit 124.

The storage unit 110 is a storage region secured in a storage device included in the diagnosis support device 100, such as the RAM 102 or the HDD 103. The storage unit 110 stores organ region classification model data 111, 2D model data 10a, and 3D model data 20a.

The organ region classification model data 111 is data indicating a trained model that performs segmentation of a region of a liver from an input tomographic image. The trained model (organ region classification model) is created by performing machine learning using a large number of tomographic images, to which labels indicating whether or not the region is a liver region are added for each pixel, as the teacher data. In a case where the organ region classification model is formed as a neural network, a weight coefficient between nodes on the neural network is included in the organ region classification model data 111.

The 2D model data 10a is data indicating the 2D model 10 described above. In a case where the 2D model 10 is formed as a neural network, a weight coefficient between nodes on the neural network is included in the 2D model data 10a. The 3D model data 20a is data indicating the 3D model 20 described above. In a case where the 3D model 20 is formed as a neural network, a weight coefficient between nodes on the neural network is included in the 3D model data 20a.

Processing of the organ region extraction unit 121, the 2D segmentation processing unit 122, the 3D segmentation processing unit 123, and the combination determination unit 124 is implemented, for example, by executing a predetermined application program by the processor 101.

By inputting each tomographic image included in the tomographic image set into the organ region classification model based on the organ region classification model data 111, the organ region extraction unit 121 extracts a liver region from the tomographic image.

The 2D segmentation processing unit 122 inputs an image of the liver region in each tomographic image included in the tomographic image set into the 2D model 10 based on the 2D model data 10a. As a result, the 2D segmentation processing unit 122 executes 2D segmentation processing for classifying whether or not each pixel is a lesion region indicating the intrahepatic bile duct dilatation for each pixel.

The 3D segmentation processing unit 123 creates volume data based on the tomographic image set, extracts the liver region from the volume data, and inputs the liver region into the 3D model 20 based on the 3D model data 20a. As a result, the 3D segmentation processing unit 123 executes 3D segmentation processing for classifying whether or not each voxel is the lesion region indicating the intrahepatic bile duct dilatation for each voxel.

The combination determination unit 124 combines a processing result of the 2D segmentation and a processing result of the 3D segmentation, calculates and outputs a final segmentation result for each tomographic image included in the tomographic image set.

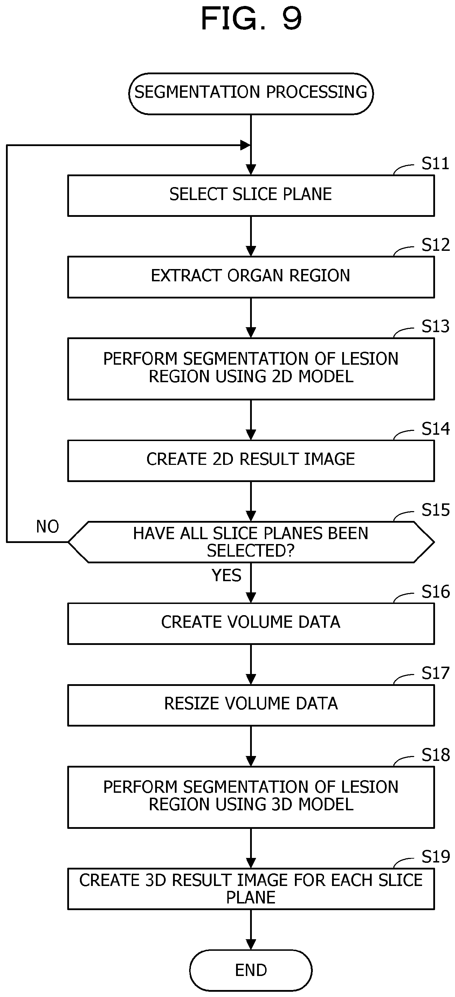

FIG. 9 is an example of a flowchart illustrating a procedure of segmentation processing using a 2D model and a 3D model.

[Step S11] The organ region extraction unit 121 selects one slice plane.

[Step S12] The organ region extraction unit 121 extracts a tomographic image corresponding to the selected slice plane, from the input tomographic image set. The organ region extraction unit 121 extracts a liver region from the tomographic image, by inputting the extracted tomographic image into the organ region classification model based on the organ region classification model data 111. With this processing, for example, in the tomographic image, a mask image in which a pixel of the liver region is set as "1" and a pixel of the other region is set as "0" is created

[Step S13] The 2D segmentation processing unit 122 performs segmentation (2D segmentation) of the lesion region, by inputting an image of the extracted liver region into the 2D model 10 based on the 2D model data 10a. As a result, each pixel of the tomographic image is classified into the lesion region and the other region.

[Step S14] The 2D segmentation processing unit 122 creates a 2D result image in which a pixel of the lesion region is set as "1" and a pixel of the other region is set as "0", in the tomographic image, based on the processing result in step S13.

[Step S15] The 2D segmentation processing unit 122 determines whether or not all the slice planes have been selected. In a case where there are unselected slice planes, the processing proceeds to step S11, and one of the unselected slice planes is selected. On the other hand, in a case where all the slice planes have been selected, the processing proceeds to step S16.

[Step S16] The 3D segmentation processing unit 123 creates volume data based on the tomographic image set.

[Step S17] The 3D segmentation processing unit 123 resizes the created volume data into a size suitable for the 3D model 20 based on the 3D model data 20a.

[Step S18] The 3D segmentation processing unit 123 performs segmentation (3D segmentation) of the lesion region, by inputting the resized volume data into the 3D model 20. As a result, each voxel of the volume data is classified into the lesion region and the other region.

[Step S19] The 3D segmentation processing unit 123 creates a 3D result image corresponding to each slice plane, based on the processing result in step S18. The 3D result image is created by resizing an image in which a pixel of the lesion region is set as "1" and a pixel of the other region is set as "0", among the images corresponding to the slice planes of the resized volume data, into a size same as the tomographic image of the input tomographic image set.

Next, a final segmentation result is calculated by the combination determination unit 124, based on a combination of the processing result of the 2D segmentation and the processing result of the 3D segmentation.

In the 2D segmentation, in a case where a shape feature of the lesion region indicating the intrahepatic bile duct dilatation appears on the axial plane, the lesion region can be detected with relatively high accuracy. However, there is a case where a region of another lesion (for example, cyst) having a shape similar to the shape appearing on the axial plane is overdetected.

On the other hand, in the 3D segmentation, a three-dimensional shape feature of a target lesion region can be captured. Therefore, there is a high possibility that only the lesion region indicating the intrahepatic bile duct dilatation can be detected, and there is a low possibility that another lesion region such as cysts is overdetected. However, as described above, in a case where the slice interval of the tomographic image set that is the creation source of the volume data input at the time of inference is larger than that at the time of training, there is a possibility that the lesion region indicating the intrahepatic bile duct dilatation cannot be accurately detected. For example, in such a case, there is a possibility that a region wider than an actual lesion region on the slice plane is detected.

Therefore, in a case where the lesion region detected through the 2D segmentation overlaps at least a part of the lesion region detected through the 3D segmentation, the combination determination unit 124 basically determines that the former lesion region is a correctly detected region. On the other hand, in a case where the former lesion region does not overlap the latter lesion region, the combination determination unit 124 determines that the former lesion region is overdetected, and rejects this lesion region without adopting this lesion region as the final detection result.

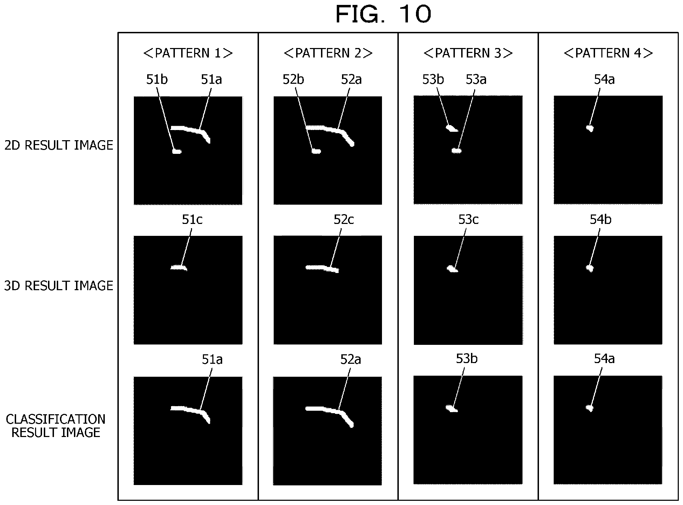

FIG. 10 is a first diagram illustrating combination determination processing. With reference to FIG. 10, a procedure of the basic combination determination processing as described above will be described.

First, the combination determination unit 124 executes 3D labeling processing, based on the 2D result image (2D result image set) created for each slice plane in step S14 in FIG. 9. Furthermore, the combination determination unit 124 executes the 3D labeling processing, based on the 3D result image (3D result image set) created for each slice plane in step S19 in FIG. 9.

In the 3D labeling processing, in a case where pixels having the same pixel value "1" are continuous on the same tomographic image and between adjacent tomographic images, a label having the same value is assigned to these pixels. Then, regions of the pixels to which the label having the same value is assigned are connected. As a result, labels having different values are assigned to respective connected regions (closed region) in which the pixels are connected, and the connected region can be identified depending on the value of the label.

The connectivity of the pixels (voxel) is determined, for example, using a six-connection method, an 18-connection method, a 26-connection method, or the like. In the six-connection method, to each of surrounding six voxels having contact with a target voxel with a voxel value "1" via a surface, a label having the same value as the target voxel is assigned, in a case where the voxels have the same voxel value "1". These voxels are connected. In the 18-connection method, to each of surrounding 18 voxels having contact with a target voxel with a voxel value "1" via a surface and a side, a label having the same value as the target voxel is assigned, in a case where the voxels have the same voxel value "1". These voxels are connected. In the 26-connection method, to each of surrounding 26 voxels having contact with a target voxel with a voxel value "1" via a surface, a side, and a vertex, a label having the same value as the target voxel is assigned, in a case where the voxels have the same voxel value "1". These voxels are connected.

Next, the combination determination unit 124 compares the connected region detected based on the 2D result image set and the connected region detected based on the 3D result image set. In a case where the former connected region overlaps at least a part of the latter connected region, the combination determination unit 124 determines that the former connected region is a correctly detected region and adopts the connected region as a final detection result.

FIG. 10 illustrates a plurality of examples, of a combination pattern between the connected region based on the 2D result image set and the connected region based on the 3D result image set. Note that the 2D result image, the 3D result image, and a classification result image illustrated in FIG. 10 for each pattern indicate images corresponding to the same slice plane. Furthermore, the classification result image is an image in which a pixel finally detected as a lesion region is set as "1" and a pixel of the other region is set as "0".

In a pattern 1, connected regions 51a and 51b are detected from the 2D result image set, and these are imaged in the 2D result image. Furthermore, a connected region 51c is detected from the 3D result image set, and this is imaged in the 3D result image of the same slice plane. Since the connected region 51a overlaps a part of the connected region 51c, the connected region 51a is adopted as the final detection result of the lesion region. On the other hand, since the connected region 51b does not overlap the connected region detected from the 3D result image set, the connected region 51b is rejected from the detection result of the lesion region.

In a pattern 2, connected regions 52a and 52b are detected from the 2D result image set, and these are imaged in the 2D result image. Furthermore, a connected region 52c is detected from the 3D result image set, and this is imaged in the 3D result image of the same slice plane. Since the connected region 52a overlaps a part of the connected region 52c, the connected region 52a is adopted as the final detection result of the lesion region. On the other hand, since the connected region 52b does not overlap the connected region detected from the 3D result image set, the connected region 52b is rejected from the detection result of the lesion region.

In a pattern 3, connected regions 53a and 53b are detected from the 2D result image set, and these are imaged in the 2D result image. Furthermore, a connected region 53c is detected from the 3D result image set, and this is imaged in the 3D result image of the same slice plane. Since the connected region 53a does not overlap the connected region detected from the 3D result image set, the connected region 53a is rejected from the detection result of the lesion region. On the other hand, since the connected region 53b overlaps a part of the connected region 53c, the connected region 53b is adopted as the final detection result of the lesion region.

In a pattern 4, a connected region 54a is detected from the 2D result image set, and this is imaged in the 2D result image. Furthermore, a connected region 54b is detected from the 3D result image set, and this is imaged in the 3D result image of the same slice plane. Since the connected region 54a overlaps a part of the connected region 54b, the connected region 54a is adopted as the final detection result of the lesion region.

According to the above procedure, only the connected region that overlaps at least a part of the connected region detected from the 3D result image set, among the connected regions detected from the 2D result image set, is adopted as the final detection result of the lesion region. The combination determination unit 124 creates a classification result image for each slice plane, based on such a detection result.

Through such combination determination processing, it is possible to prevent occurrence of overdetection by the 2D segmentation, and the segmentation accuracy can be improved. In other words, the connected region detected by the 2D segmentation may include an erroneously detected region. On the other hand, with the 3D segmentation, detection processing for accurately capturing a three-dimensional shape of the lesion region can be expected. However, there is a possibility that detection accuracy is lowered, depending on the number of slices of the input tomographic image set. On the other hand, in a case where the connected region detected by the 2D segmentation overlaps at least a part of the connected region detected by the 3D segmentation, it is estimated that reliability of the former connected region is higher. Therefore, with such processing, the segmentation accuracy can be improved.

Furthermore, the final detection result is selected from among the detection results by the 2D segmentation. As a result, regardless of whether the number of slices of the tomographic image set input at the time of inference matches or does not match the number of slices of the tomographic image set that is the creation source of the volume data used at the time of training, it is possible to obtain an improving effect of the segmentation accuracy described above.

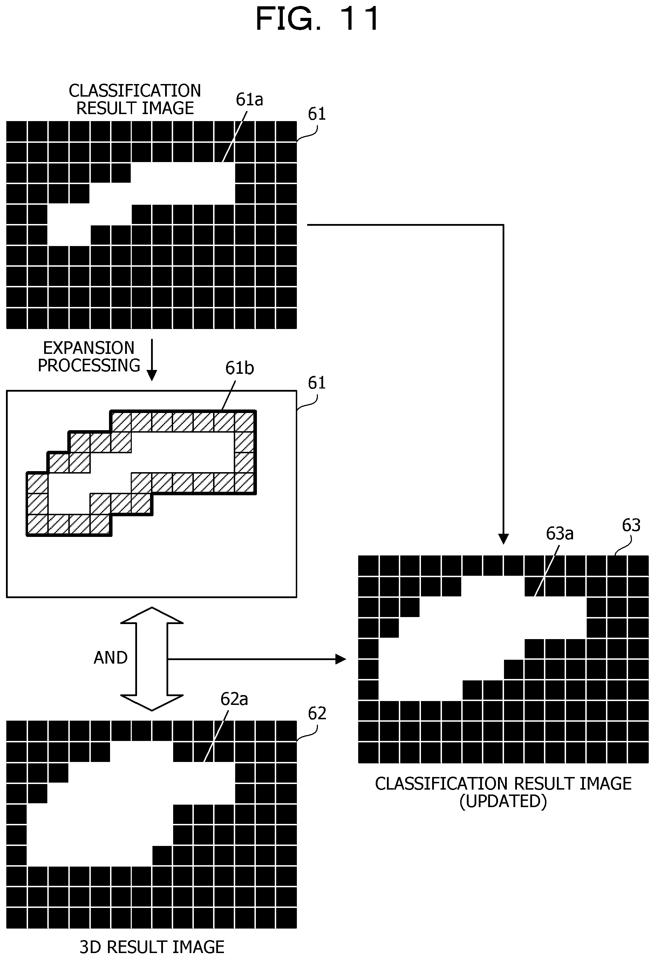

FIG. 11 is a second diagram illustrating the combination determination processing. After executing the processing described with reference to FIG. 10, the combination determination unit 124 additionally executes the following processing.

The combination determination unit 124 selects a connected region with the largest volume, from among the connected regions detected from the 3D result image set. The combination determination unit 124 selects a slice plane including the selected connected region and determines whether or not a lesion region exists in a classification result image corresponding to the slice plane.

In a classification result image 61 illustrated in FIG. 11, a lesion region 61a exists. In this case, the combination determination unit 124 executes expansion processing for enlarging the periphery of the lesion region 61a. In the example in FIG. 11, by executing the expansion processing for adding a region for one pixel having contact with an outer edge of the lesion region 61a to the lesion region 61a, an expansion region 61b is set.

The combination determination unit 124 performs an AND (logical product) operation of the expansion region 61b and a lesion region 62a included in a 3D result image 62 on the same slice plane. The combination determination unit 124 adds a pixel that is determined as an overlap between the expansion region 61b and the lesion region 62a, as a result of the AND operation to the classification result image, as the lesion region. As actual processing, a pixel value of a pixel of which a pixel value is "0" in the classification result image 61, among the pixels determined as the overlap between the expansion region 61b and the lesion region 62a is updated to "1". In FIG. 11, in a classification result image 63 in which the pixel value is updated in this way, a lesion region 63a enlarged from the lesion region 61a is set.

In this example, the lesion region 61a included in the classification result image 61 indicates a lesion region with high reliability, from among the lesion regions detected from the 2D result image set. A lesion region detected from the 3D result image set, positioned around such a lesion region with high reliability is also estimated to have high reliability as a detection result. Therefore, by incorporating the latter lesion region into the final result, it is possible to more accurately detect a lesion.

Note that the number of expansions (the number of pixels enlarged around lesion region) in the expansion processing can be arbitrarily set. As the number of expansions increases, a reproduction rate increases, and a matching rate tends to decrease.

On the other hand, although not illustrated, in a case where no lesion region exists in the classification result image corresponding to the slice plane including the connected region with the largest volume, the combination determination unit 124 determines that the lesion region included in the 3D result image of the same slice plane as a correct detection result and adds this lesion region to the classification result image. As actual processing, a pixel value of the pixel of the lesion region included in the 3D result image, among the pixels of the classification result image, is updated from "0" to "1".

There is a high possibility that a connected region with a small volume, among the connected regions detected from the 3D result image set, is erroneously detected. However, there is a high possibility that a connected region with a large volume is accurately detected. According to the processing described above, by detecting the pixel of the classification result image included in the latter connected region as the lesion region, it is possible to prevent occurrence of detection omission of the lesion region by the 2D segmentation and to improve the segmentation accuracy.

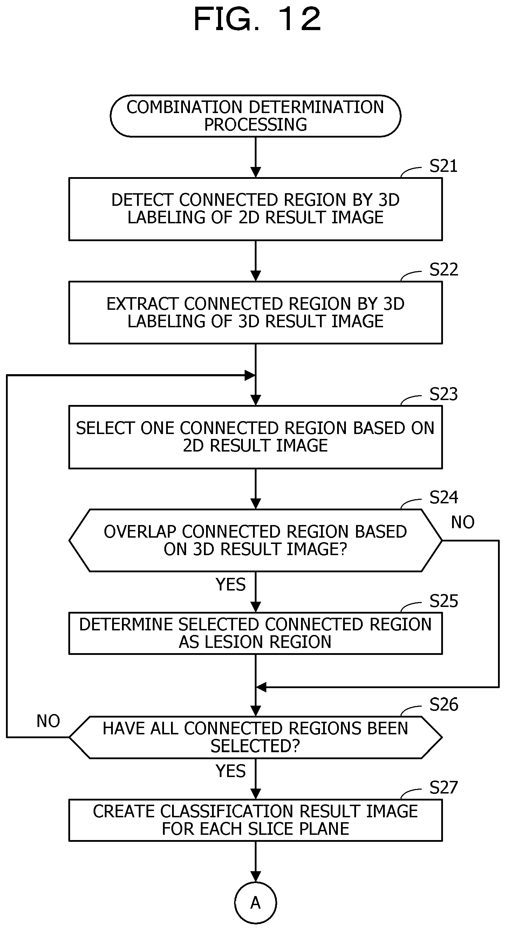

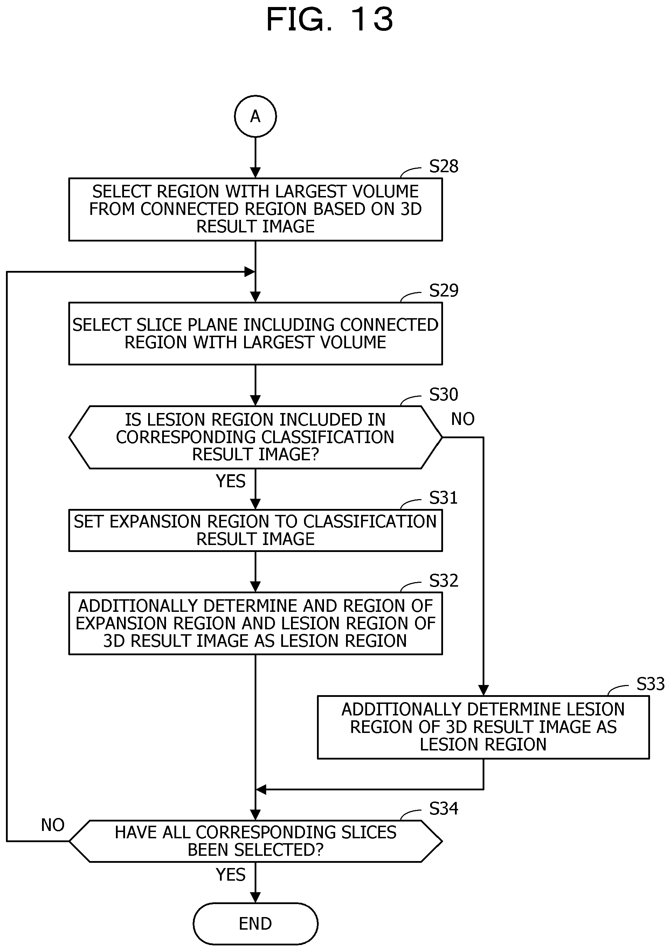

FIGs. 12 and 13 are examples of a flowchart illustrating a procedure of the combination determination processing.

[Step S21] The combination determination unit 124 executes the 3D labeling processing based on the 2D result image set created by the 2D segmentation and detects a connected region of a lesion in a three-dimensional space.

[Step S22] The combination determination unit 124 executes the 3D labeling processing based on the 3D result image set created by the 3D segmentation and detects the connected region of the lesion in the three-dimensional space.

[Step S23] The combination determination unit 124 selects one connected region based on the 2D result image set.

[Step S24] The combination determination unit 124 determines whether or not the selected connected region overlaps at least a part of the connected region detected based on the 3D result image set. In a case where the former connected region overlaps at least a part of the latter connected region, the processing proceeds to step S25, and in a case where the former connected region does not overlap any part of the latter connected region, the processing proceeds to step S26.

[Step S25] The combination determination unit 124 determines that the connected region selected in step S23 as a correct lesion region.

[Step S26] The combination determination unit 124 determines whether or not all the connected regions detected based on the 2D result image set have been selected. In a case where there is an unselected connected region, the processing proceeds to step S23, and one of the unselected connected regions is selected. On the other hand, in a case where all the connected regions have been selected, the processing proceeds to step S27.

[Step S27] The combination determination unit 124 creates a classification result image corresponding to each slice plane, based on the determination result in step S25. The classification result image is an image in which a pixel included in a region determined as the lesion region in step S25 is set as "1" and another pixel is set as "0", in a tomographic image of the corresponding slice plane.

[Step S28] The combination determination unit 124 selects a connected region with the largest volume, from among the connected regions detected based on the 3D result image set.

[Step S29] The combination determination unit 124 selects a slice plane including the connected region selected in step S28.

[Step S30] The combination determination unit 124 determines whether or not a lesion region exists in a classification result image corresponding to the selected slice plane. In a case where the lesion region exists, the processing proceeds to step S31, and in a case where no lesion region exists, the processing proceeds to step S33.

[Step S31] The combination determination unit 124 executes the expansion processing on the lesion region in the classification result image and sets an expansion region enlarged from the lesion region.

[Step S32] The combination determination unit 124 performs an AND operation of the set expansion region and the lesion region existing in the 3D result image on the same slice plane. As a result of the AND operation, the combination determination unit 124 additionally determines that the pixel determined as an overlap between the former expansion region and the latter lesion region as a correct lesion region. The combination determination unit 124 updates a pixel value of the pixel determined as an overlap through the AND operation, among the pixels having the pixel value "0" in the classification result image, to "1".

[Step S33] The combination determination unit 124 additionally determines that the lesion region existing in the 3D result image (region corresponding to connected region with largest volume) as a correct lesion region. The combination determination unit 124 updates a pixel value of the pixel determined as the lesion region from the 3D result image, from among the pixels having the pixel value "0" in the classification result image to "1".

[Step S34] The combination determination unit 124 determines whether or not all the slice planes including the connected region selected in step S28 have been selected. In a case where there are unselected slice planes, the processing proceeds to step S29, and one of the unselected slice planes is selected. On the other hand, in a case where all the corresponding slice planes have been selected, the combination determination processing ends, and the classification result image for each slice plane at this time is output.

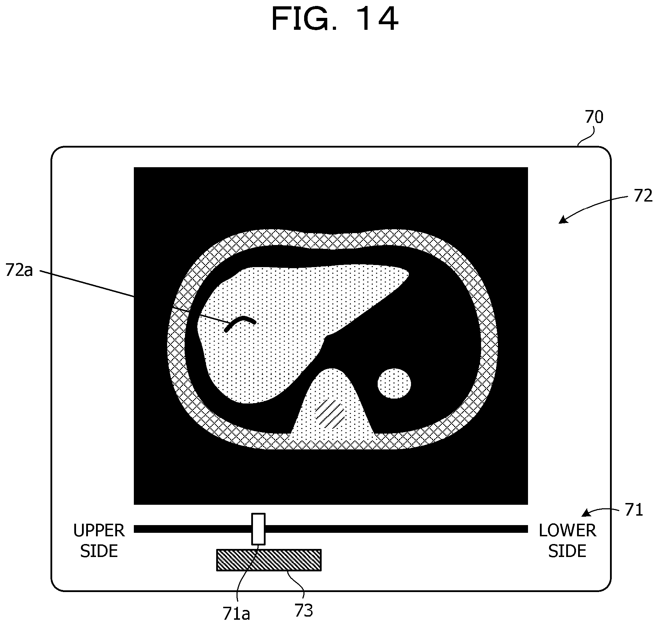

FIG. 14 is a diagram illustrating a screen display example of a detection result of a lesion region. The diagnosis support device 100 can output information indicating the detection result of the lesion region, using the classification result image created for each slice plane. For example, the diagnosis support device 100 can cause a display device to display a result display screen as illustrated in FIG. 14. A result display screen 70 includes a slice selection portion 71, a tomographic image display portion 72, and a lesion detection region display portion 73.

In the slice selection portion 71, a slice plane of a tomographic image displayed in the tomographic image display portion 72 can be selected by moving a handle 71a on a slider. A tomographic image corresponding to the slice plane selected in the slice selection portion 71 is displayed in the tomographic image display portion 72. On this tomographic image, a lesion region 72a is superimposed and displayed, based on a classification result image corresponding to the same slice plane. In other words, the lesion region 72a indicates a region with a pixel value "1" in the classification result image.

The lesion detection region display portion 73 indicates a range of the slice plane in which the lesion region is detected (slice plane corresponding to classification result image in which pixel with pixel value "1" exists), in a movable region of the handle 71a in the slice selection portion 71. With this display of the lesion detection region display portion 73, it is possible for a user to easily recognize the slice planes in which the lesion region is detected, to quickly display the tomographic image corresponding to these slice planes, and to confirm the lesion region in the tomographic image.

Note that, according to the processing described above of the diagnosis support device 100, regardless of whether the number of slices of the tomographic image set input at the time of inference matches or does not match the number of slices of the tomographic image set that is the creation source of the volume data used at the time of training, the segmentation accuracy can be improved. In addition to such a viewpoint, each piece of the volume data used as the teacher data at the time of training the 3D model 20 is not necessarily limited to be created based on the tomographic image set having the same slice interval. By performing machine learning using the volume data based on the tomographic image set with different slice intervals, there is a possibility that the segmentation accuracy of the created 3D model 20 is lowered. By combining the segmentation result by such a 3D model 20 and the segmentation result by the 2D model 10 and outputting the final result, the diagnosis support device 100 described above can improve the segmentation accuracy than that in a case where only the former result is used.

Furthermore, in the second embodiment described above, an example has been described where the liver is applied as the organ and the intrahepatic bile duct dilatation is applied as the lesion to be detected. However, the organ and the lesion are not limited to the examples described above, and the technology described above can be applied to detect another lesion in another organ.

### <Modification of Second Embodiment>

Next, a modification in which a part of the processing of the diagnosis support device 100 according to the second embodiment is modified will be described. In this modification, a case will be described where a spherical lesion region, in particular, a tumor region is detected as a specific lesion.

For example, a region of a tumor that is darker than a surrounding region can be detected using a trained model created through machine learning. However, there is a case where a normal organ region (organ region where tumor does not exist) is overdetected. When the number of times of overdetection is large, it is necessary to confirm an unnecessary tomographic image in the tomographic image set for interpretation of radiogram, a time needed for confirmation increases, and work efficiency is lowered. On the other hand, in image recognition using machine learning, typically, overdetection and detection omission have a trade-off relationship, and it is not easy to prevent occurrence of the overdetection without increasing the detection omissions.

Therefore, by finally detecting a lesion region from a combination of a result of 2D segmentation and a result of 3D segmentation, the occurrence of the overdetection and the detection omission is prevented, and the detection accuracy of the lesion region is improved.

The 2D segmentation of the tumor region is highly sensitive with respect to colors. Therefore, in a case where a feature of the tumor region that is darker than surroundings is machine-learned, the sensitivity with respect to the colors becomes stronger, and it is easier to overdetect a dark circular normal organ region. For example, since a blood vessel appears as a circle in a 2D image, there is a case where the blood vessel is erroneously detected as a tumor.

On the other hand, since a feature of a three-dimensional shape is machine-learned in the 3D segmentation, a possibility of overdetecting the blood vessel is low. However, depending on a state of shadow of an organ, there is a possibility that the normal organ region having a similar shape to a tumor is overdetected.

In this way, in the detection of the spherical lesion, there is a possibility that overdetection occurs in both of the 2D segmentation and the 3D segmentation. However, tendencies of the overdetection are different. Therefore, by determining that the overdetection occurs in a case where the lesion region detected by the 2D segmentation does not overlap the lesion region detected by the 3D segmentation, it is possible to prevent the occurrence of the overdetection.

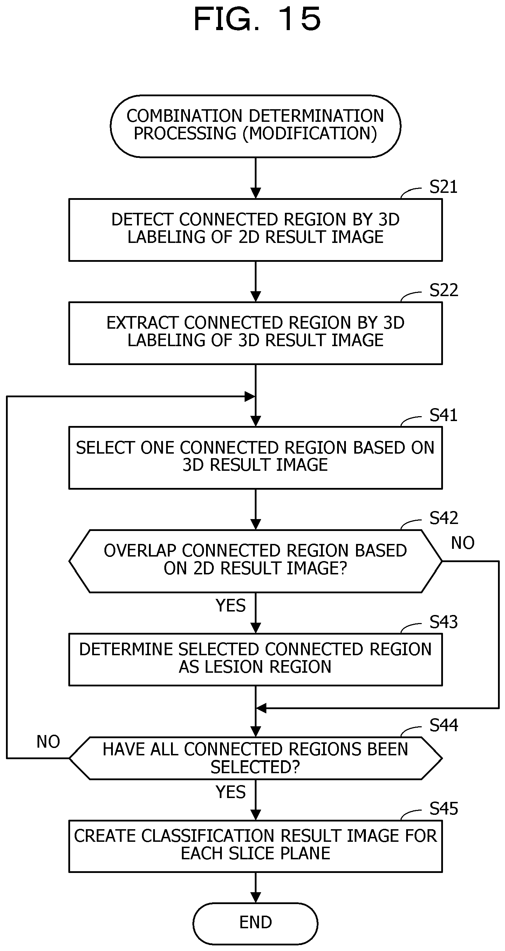

In this modification, the diagnosis support device 100 executes 2D and 3D segmentation processing similar to that in FIG. 9 as setting the lesion to be detected as a tumor. Thereafter, the following processing illustrated in FIG. 15 is executed.

FIG. 15 is an example of a flowchart illustrating a procedure of combination determination processing according to the modification. In FIG. 15, a processing step in which processing as in FIG. 12 is executed is denoted with the same reference numeral.

The diagnosis support device 100 executes processing in steps S21 and S22 using the created 2D result image set and 3D result image set. Thereafter, processing in steps S41 to S45 illustrated in FIG. 15 is executed.

[Step S41] The combination determination unit 124 selects one connected region based on the 3D result image set.

[Step S42] The combination determination unit 124 determines whether or not the selected connected region overlaps at least a part of the connected region detected based on the 2D result image set. In a case where the former connected region overlaps at least a part of the latter connected region, the processing proceeds to step S43, and in a case where the former connected region does not overlap any part of the latter connected region, the processing proceeds to step S44.

[Step S43] The combination determination unit 124 determines that the connected region selected in step S41 is a correct lesion region (tumor region).

[Step S44] The combination determination unit 124 determines whether or not all the connected regions detected based on the 3D result image set have been selected. In a case where there is an unselected connected region, the processing proceeds to step S41, and one of the unselected connected regions is selected. On the other hand, in a case where all the connected regions have been selected, the processing proceeds to step S45.

[Step S45] The combination determination unit 124 creates a classification result image corresponding to each slice plane, based on the determination result in step S43. The classification result image is an image in which a pixel included in a region determined as the lesion region in step S43 is set as "1" and another pixel is set as "0", in a tomographic image of the corresponding slice plane.

In the processing described above, since the detection accuracy of the tumor region by the 3D segmentation is higher than that of the 2D segmentation, in a case where the connected region by the former overlaps at least a part of the connected region by the latter, the connected region by the former is determined as a correct lesion region. As a result, it is possible to prevent the occurrence of the overdetection and the detection omission and to improve the detection accuracy of the lesion region.

Note that, the processing functions of the device (for example, lesion detection device 1 and diagnosis support device 100) described in each embodiment described above can be implemented by a computer. In that case, a program describing the processing content of the functions to be held by each device is provided, and the processing functions described above are implemented on the computer by execution of the program on the computer. The program describing the processing content may be recorded in a computer-readable recording medium. Examples of the computer-readable recording medium include a magnetic storage device, an optical disk, a semiconductor memory, or the like. Examples of the magnetic storage device include a hard disk drive (HDD), a magnetic tape, or the like. Examples of the optical disk include a compact disc (CD), a digital versatile disc (DVD), a Blu-ray disc (BD, registered trademark), or the like.

In a case where the program is to be distributed, for example, portable recording media such as DVDs or CDs in which the program is recorded are sold. Furthermore, it is also possible to store the program in a storage device of a server computer, and transfer the program from the server computer to another computer via a network.

The computer that executes the program stores, for example, the program recorded in the portable recording medium or the program transferred from the server computer in its own storage device. Then, the computer reads the program from its own storage device, and executes processing according to the program. Note that the computer may read the program directly from the portable recording medium and execute the processing according to the program. Furthermore, the computer may sequentially execute processing according to the received program each time the program is transferred from the server computer coupled via the network.

## Claims

1. A lesion detection method for a computer to execute a process comprising:
detecting a first lesion region that indicates a certain lesion from each of a plurality of tomographic images (3a-3c) obtained by imaging an inside of a human body, by using a first machine learning model (5a) that detects a lesion region from an input image that has image data of a two-dimensional space;
detecting a second lesion region that indicates the certain lesion from three-dimensional volume data generated based on the plurality of tomographic images, by using a second machine learning model (5b) that detects a lesion region from input volume data that has image data of a three-dimensional space; and
detecting a third lesion region that indicates the certain lesion from each of the plurality of tomographic images, based on an overlapping state between the first lesion region and the second lesion region,
**characterised in that** the detecting the third lesion region includes:
assigning a label having a same value to pixels which have a same pixel value and are continuous on a same tomographic image and between adjacent tomographic images and connecting regions of the pixels to which the label having the same value is assigned to acquire a closed region identified depending on the value of the label,
detecting a first closed region that indicates the certain lesion in the three-dimensional space, based on the detecting the first lesion region;
detecting a second closed region that indicates the certain lesion in the three-dimensional space, based on the detecting the second lesion region; and
detecting the third lesion region based on an overlapping state between the first closed region and the second closed region.

2. The lesion detection method according to claim 1, wherein the process further comprising
when the first lesion region overlaps at least a part of the second lesion region, detecting the first lesion region as the third lesion region.

3. The lesion detection method according to claim 1, wherein the detecting the third lesion region includes
detecting a region that corresponds to the first closed region in each of the plurality of tomographic images as the third lesion region, when the first closed region overlaps at least a part of the second closed region.

4. The lesion detection method according to claim 3, wherein the detecting the third lesion region includes:
selecting a third closed region with a best volume from among a plurality of the second closed regions detected from the three-dimensional volume data;
selecting a first tomographic image that includes the third closed region, from among the plurality of tomographic images; and
adding a region that overlaps between an enlarged region obtained by enlarging the third lesion region in the first tomographic image and a region that corresponds to the second lesion region in the first tomographic image as the third lesion region in the first tomographic image.

5. The lesion detection method according to claim 4, wherein the detecting the third lesion region includes
detecting a region that corresponds to the third closed region in the first tomographic image as the third lesion region in the first tomographic image when the third lesion region is not detected from the selected first tomographic image.

6. A non-transitory computer-readable storage medium storing a lesion detection program that causes at least one computer to execute a process, the process comprising:
detecting a first lesion region that indicates a certain lesion from each of a plurality of tomographic images (3a-3c) obtained by imaging an inside of a human body, by using a first machine learning model (5a) that detects a lesion region from an input image that has image data of a two-dimensional space;
detecting a second lesion region that indicates the certain lesion from three-dimensional volume data generated based on the plurality of tomographic images, by using a second machine learning model (5b) that detects a lesion region from input volume data that has image data of a three-dimensional space; and
detecting a third lesion region that indicates the certain lesion from each of the plurality of tomographic images, based on an overlapping state between the first lesion region and the second lesion region,
chracterised in that the detecting the third lesion region includes:
assigning a label having a same value to pixels which have a same pixel value and are continuous on a same tomographic image and between adjacent tomographic images and connecting regions of the pixels to which the label having the same value is assigned to acquire a closed region identified depending on the value of the label,
detecting a first closed region that indicates the certain lesion in the three-dimensional space, based on the detecting the first lesion region;
detecting a second closed region that indicates the certain lesion in the three-dimensional space, based on the detecting the second lesion region; and
detecting the third lesion region based on an overlapping state between the first closed region and the second closed region.

7. The non-transitory computer-readable storage medium according to claim 6, wherein the process further comprising
when the first lesion region overlaps at least a part of the second lesion region, detecting the first lesion region as the third lesion region.

8. The non-transitory computer-readable storage medium according to claim 6, wherein the detecting the third lesion region includes
detecting a region that corresponds to the first closed region in each of the plurality of tomographic images as the third lesion region, when the first closed region overlaps at least a part of the second closed region.

9. The non-transitory computer-readable storage medium according to claim 8, wherein the detecting the third lesion region includes:
selecting a third closed region with a best volume from among a plurality of the second closed regions detected from the three-dimensional volume data;
selecting a first tomographic image that includes the third closed region, from among the plurality of tomographic images; and
adding a region that overlaps between an enlarged region obtained by enlarging the third lesion region in the first tomographic image and a region that corresponds to the second lesion region in the first tomographic image as the third lesion region in the first tomographic image.

10. The non-transitory computer-readable storage medium according to claim 9, wherein the detecting the third lesion region includes
detecting a region that corresponds to the third closed region in the first tomographic image as the third lesion region in the first tomographic image when the third lesion region is not detected from the selected first tomographic image.

## Patentansprüche

1. Läsionsdetektionsverfahren für einen Computer zum Ausführen eines Prozesses, umfassend:
Detektieren eines ersten Läsionsgebiets, das eine bestimmte Läsion darstellt, aus jedem einer Vielzahl von tomographischen Bildern (3a-3c), die durch Bildgebung eines Inneren eines menschlichen Körpers erhalten wurden, unter Verwendung eines ersten maschinellen Lernmodells (5a), das ein Läsionsgebiet aus einem Eingabebild detektiert, das Bilddaten eines zweidimensionalen Raums aufweist;
Detektieren eines zweiten Läsionsgebiets, das die bestimmte Läsion aus dreidimensionalen Volumendaten darstellt, die basierend auf der Vielzahl von tomographischen Bildern erzeugt wurden, unter Verwendung eines zweiten maschinellen Lernmodells (5b), das ein Läsionsgebiet aus Eingabevolumendaten detektiert, die Bilddaten eines dreidimensionalen Raums aufweisen; und
Detektieren eines dritten Läsionsgebiets, das die bestimmte Läsion aus jedem der Vielzahl von tomographischen Bildern darstellt, basierend auf einem überlappenden Zustand zwischen dem ersten Läsionsgebiet und dem zweiten Läsionsgebiet,
**dadurch gekennzeichnet, dass** das Detektieren des dritten Läsionsgebiets Folgendes einschließt:
Zuweisen einer Kennzeichnung, die einen gleichen Wert aufweist, zu Pixeln, die einen gleichen Pixelwert aufweisen und auf einem gleichen tomographischen Bild und zwischen angrenzenden tomographischen Bildern zusammenhängend sind, und Verbinden von Gebieten der Pixel, denen die Kennzeichnung, die den gleichen Wert aufweist, zugewiesen ist, um ein geschlossenes Gebiet zu erfassen, das abhängig von dem Wert der Kennzeichnung identifiziert wird,
Detektieren eines ersten geschlossenen Gebiets, das die bestimmte Läsion in dem dreidimensionalen Raum darstellt, basierend auf dem Detektieren des ersten Läsionsgebiets;
Detektieren eines zweiten geschlossenen Gebiets, das die bestimmte Läsion in dem dreidimensionalen Raum darstellt, basierend auf dem Detektieren des zweiten Läsionsgebiets; und
Detektieren des dritten Läsionsgebiets basierend auf einem überlappenden Zustand zwischen dem ersten geschlossenen Gebiet und dem zweiten geschlossenen Gebiet.

2. Läsionsdetektionsverfahren nach Anspruch 1, wobei der Prozess weiter umfasst, wenn das erste Läsionsgebiet mindestens einen Teil des zweiten Läsionsgebiets überlappt, Detektieren des ersten Läsionsgebiets als das dritte Läsionsgebiet.

3. Läsionsdetektionsverfahren nach Anspruch 1, wobei das Detektieren des dritten Läsionsgebiets einschließt
Detektieren eines Gebiets, das dem ersten geschlossenen Gebiet in jedem der Vielzahl von tomographischen Bildern entspricht, als das dritte Läsionsgebiet, wenn das erste geschlossene Gebiet mindestens einen Teil des zweiten geschlossenen Gebiets überlappt.

4. Läsionsdetektionsverfahren nach Anspruch 3, wobei das Detektieren des dritten Läsionsgebiets einschließt:
Auswählen eines dritten geschlossenen Gebiets mit einem besten Volumen aus einer Vielzahl von zweiten geschlossenen Gebieten, die aus den dreidimensionalen Volumendaten detektiert wurden;
Auswählen eines ersten tomographischen Bildes, das das dritte geschlossene Gebiet einschließt, aus der Vielzahl von tomographischen Bildern; und
Hinzufügen eines Gebiets, das sich mit einem vergrößerten Gebiet überlappt, das durch Vergrößern des dritten Läsionsgebiets im ersten tomographischen Bild erhalten wurde, und eines Gebiets, das dem zweiten Läsionsgebiet im ersten tomographischen Bild entspricht, als das dritte Läsionsgebiet im ersten tomographischen Bild.

5. Läsionsdetektionsverfahren nach Anspruch 4, wobei das Detektieren des dritten Läsionsgebiets einschließt
Detektieren eines Gebiets, das dem dritten geschlossenen Gebiet in dem ersten tomographischen Bild entspricht, als das dritte Läsionsgebiet in dem ersten tomographischen Bild, wenn das dritte Läsionsgebiet in dem ausgewählten ersten tomographischen Bild nicht detektiert wird.

6. Nicht-transitorisches, computerlesbares Speichermedium, das ein Läsionsdetektionsprogramm speichert, das mindestens einen Computer dazu veranlasst, einen Prozess auszuführen, wobei der Prozess Folgendes umfasst:
Detektieren eines ersten Läsionsgebiets, das eine bestimmte Läsion darstellt, aus jedem einer Vielzahl von tomographischen Bildern (3a-3c), die durch Bildgebung eines Inneren eines menschlichen Körpers erhalten wurden, unter Verwendung eines ersten maschinellen Lernmodells (5a), das ein Läsionsgebiet aus einem Eingabebild detektiert, das Bilddaten eines zweidimensionalen Raums aufweist;
Detektieren eines zweiten Läsionsgebiets, das die bestimmte Läsion aus dreidimensionalen Volumendaten darstellt, die basierend auf der Vielzahl von tomographischen Bildern erzeugt wurden, unter Verwendung eines zweiten maschinellen Lernmodells (5b), das ein Läsionsgebiet aus Eingabevolumendaten detektiert, die Bilddaten eines dreidimensionalen Raums aufweisen; und
Detektieren eines dritten Läsionsgebiets, das die bestimmte Läsion aus jedem der Vielzahl von tomographischen Bildern darstellt, basierend auf einem überlappenden Zustand zwischen dem ersten Läsionsgebiet und dem zweiten Läsionsgebiet,
**dadurch gekennzeichnet, dass** das Detektieren des dritten Läsionsgebiet Folgendes einschließt:
Zuweisen einer Kennzeichnung, die einen gleichen Wert aufweist, zu Pixeln, die einen gleichen Pixelwert aufweisen und auf einem gleichen tomographischen Bild und zwischen angrenzenden tomographischen Bildern zusammenhängend sind, und Verbinden von Gebieten der Pixel, denen die Kennzeichnung, die den gleichen Wert aufweist, zugewiesen ist, um ein geschlossenes Gebiet zu erfassen, das abhängig von dem Wert der Kennzeichnung identifiziert wird,
Detektieren eines ersten geschlossenen Gebiets, das die bestimmte Läsion in dem dreidimensionalen Raum darstellt, basierend auf dem Detektieren des ersten Läsionsgebiets;
Detektieren eines zweiten geschlossenen Gebiets, das die bestimmte Läsion in dem dreidimensionalen Raum darstellt, basierend auf dem Detektieren des zweiten Läsionsgebiets; und
Detektieren des dritten Läsionsgebiets basierend auf einem überlappenden Zustand zwischen dem ersten geschlossenen Gebiet und dem zweiten geschlossenen Gebiet.

7. Nicht-transitorisches, computerlesbares Speichermedium nach Anspruch 6, wobei der Prozess weiter umfasst
wenn das erste Läsionsgebiet mindestens einen Teil des zweiten Läsionsgebiets überlappt, Detektieren des ersten Läsionsgebiets als das dritte Läsionsgebiet.

8. Nicht-transitorisches, computerlesbares Speichermedium nach Anspruch 6, wobei das Detektieren des dritten Läsionsgebiets einschließt
Detektieren eines Gebiets, das dem ersten geschlossenen Gebiet in jedem der Vielzahl von tomographischen Bildern entspricht, als das dritte Läsionsgebiet, wenn das erste geschlossene Gebiet mindestens einen Teil des zweiten geschlossenen Gebiets überlappt.

9. Nicht-transitorisches, computerlesbares Speichermedium nach Anspruch 8, wobei das Detektieren des dritten Läsionsgebiets einschließt:
Auswählen eines dritten geschlossenen Gebiets mit einem besten Volumen aus einer Vielzahl von zweiten geschlossenen Gebieten, die aus den dreidimensionalen Volumendaten detektiert wurden;
Auswählen eines ersten tomographischen Bildes, das das dritte geschlossene Gebiet einschließt, aus der Vielzahl von tomographischen Bildern; und
Hinzufügen eines Gebiets, das sich mit einem vergrößerten Gebiet überlappt, das durch Vergrößern des dritten Läsionsgebiets im ersten tomographischen Bild erhalten wurde, und eines Gebiets, das dem zweiten Läsionsgebiet im ersten tomographischen Bild entspricht, als das dritte Läsionsgebiet im ersten tomographischen Bild.

10. Nicht-transitorisches, computerlesbares Speichermedium nach Anspruch 9, wobei das Detektieren des dritten Läsionsgebiets einschließt
Detektieren eines Gebiets, das dem dritten geschlossenen Gebiet in dem ersten tomographischen Bild entspricht, als das dritte Läsionsgebiet in dem ersten tomographischen Bild, wenn das dritte Läsionsgebiet nicht in dem ausgewählten ersten tomographischen Bild detektiert wird.

## Revendications

1. Procédé de détection de lésion pour qu'un ordinateur exécute un processus comprenant :
la détection d'une première région de lésion qui indique une lésion certaine à partir de chacune d'une pluralité d'images tomographiques (3a-3c) obtenues par l'imagerie d'un intérieur d'un corps humain, en utilisant un premier modèle d'apprentissage automatique (5a) qui détecte une région de lésion à partir d'une image d'entrée qui présente des données d'image d'un espace bidimensionnel ;
la détection d'une deuxième région de lésion qui indique la lésion certaine à partir de données de volume tridimensionnelles générées sur la base de la pluralité d'images tomographiques, en utilisant un deuxième modèle d'apprentissage automatique (5b) qui détecte une région de lésion à partir de données de volume d'entrée qui présentent des données d'image d'un espace tridimensionnel ; et
la détection d'une troisième région de lésion qui indique la lésion certaine à partir de chacune de la pluralité d'images tomographiques, sur la base d'un état de chevauchement entre la première région de lésion et la deuxième région de lésion,
**caractérisé en ce que** la détection de la troisième région de lésion inclut :
l'attribution d'une étiquette présentant une même valeur à des pixels qui présentent une même valeur de pixel et sont continus sur une même image tomographique et entre des images tomographiques adjacentes et la liaison de régions des pixels auxquels l'étiquette présentant la même valeur est attribuée pour acquérir une région fermée identifiée en fonction de la valeur de l'étiquette,
la détection d'une première région fermée qui indique la lésion certaine dans l'espace tridimensionnel, sur la base de la détection de la première région de lésion ;
la détection d'une deuxième région fermée qui indique la lésion certaine dans l'espace tridimensionnel, sur la base de la détection de la deuxième région de lésion ; et
la détection de la troisième région de lésion sur la base d'un état de chevauchement entre la première région fermée et la deuxième région fermée.

2. Procédé de détection de lésion selon la revendication 1, dans lequel le processus comprend en outre, lorsque la première région de lésion chevauche au moins une partie de la deuxième région de lésion, la détection de la première région de lésion comme étant la troisième région de lésion.

3. Procédé de détection de lésion selon la revendication 1, dans lequel la détection de la troisième région de lésion inclut
la détection d'une région qui correspond à la première région fermée dans chacune de la pluralité d'images tomographiques comme étant la troisième région de lésion, lorsque la première région fermée chevauche au moins une partie de la deuxième région fermée.

4. Procédé de détection de lésion selon la revendication 3, dans lequel la détection de la troisième région de lésion inclut :
la sélection d'une troisième région fermée dotée d'un meilleur volume parmi une pluralité des deuxièmes régions fermées détectées à partir des données de volume tridimensionnelles ;
la sélection d'une première image tomographique qui inclut la troisième région fermée, parmi la pluralité d'images tomographiques ; et
l'ajout d'une région qui chevauche entre une région agrandie obtenue par l'agrandissement de la troisième région de lésion dans la première image tomographique et une région qui correspond à la deuxième région de lésion dans la première image tomographique comme étant la troisième région de lésion dans la première image tomographique.

5. Procédé de détection de lésion selon la revendication 4, dans lequel la détection de la troisième région de lésion inclut
la détection d'une région qui correspond à la troisième région fermée dans la première image tomographique comme étant la troisième région de lésion dans la première image tomographique lorsque la troisième région de lésion n'est pas détectée à partir de la première image tomographique sélectionnée.

6. Support de stockage non transitoire lisible par ordinateur stockant un programme de détection de lésion qui amène au moins un ordinateur à mettre en œuvre un processus, le processus comprenant :
la détection d'une première région de lésion qui indique une lésion certaine à partir de chacune d'une pluralité d'images tomographiques (3a-3c) obtenues par l'imagerie d'un intérieur d'un corps humain, en utilisant un premier modèle d'apprentissage automatique (5a) qui détecte une région de lésion à partir d'une image d'entrée qui présente des données d'image d'un espace bidimensionnel ;
la détection d'une deuxième région de lésion qui indique la lésion certaine à partir de données de volume tridimensionnelles générées sur la base de la pluralité d'images tomographiques, en utilisant un deuxième modèle d'apprentissage automatique (5b) qui détecte une région de lésion à partir de données de volume d'entrée qui présentent des données d'image d'un espace tridimensionnel ; et
la détection d'une troisième région de lésion qui indique la lésion certaine à partir de chacune de la pluralité d'images tomographiques, sur la base d'un état de chevauchement entre la première région de lésion et la deuxième région de lésion,
**caractérisé en ce que** la détection de la troisième région de lésion inclut :
l'attribution d'une étiquette présentant une même valeur à des pixels qui présentent une même valeur de pixel et sont continus sur une même image tomographique et entre des images tomographiques adjacentes et la liaison de régions des pixels auxquels l'étiquette présentant la même valeur est attribuée pour acquérir une région fermée identifiée en fonction de la valeur de l'étiquette,
la détection d'une première région fermée qui indique la lésion certaine dans l'espace tridimensionnel, sur la base de la détection de la première région de lésion ;
la détection d'une deuxième région fermée qui indique la lésion certaine dans l'espace tridimensionnel, sur la base de la détection de la deuxième région de lésion ; et
la détection de la troisième région de lésion sur la base d'un état de chevauchement entre la première région fermée et la deuxième région fermée.

7. Support de stockage non transitoire lisible par ordinateur selon la revendication 6, dans lequel le processus comprend en outre
lorsque la première région de lésion chevauche au moins une partie de la deuxième région de lésion, la détection de la première région de lésion comme étant la troisième région de lésion.

8. Support de stockage non transitoire lisible par ordinateur selon la revendication 6, dans lequel la détection de la troisième région de lésion inclut
la détection d'une région qui correspond à la première région fermée dans chacune de la pluralité d'images tomographiques comme étant la troisième région de lésion, lorsque la première région fermée chevauche au moins une partie de la deuxième région fermée.

9. Support de stockage non transitoire lisible par ordinateur selon la revendication 8, dans lequel la détection de la troisième région de lésion inclut :
la sélection d'une troisième région fermée dotée d'un meilleur volume parmi une pluralité des deuxièmes régions fermées détectées à partir des données de volume tridimensionnelles ;
la sélection d'une première image tomographique qui inclut la troisième région fermée, parmi la pluralité d'images tomographiques ; et
l'ajout d'une région qui chevauche entre une région agrandie obtenue par l'agrandissement de la troisième région de lésion dans la première image tomographique et une région qui correspond à la deuxième région de lésion dans la première image tomographique comme étant la troisième région de lésion dans la première image tomographique.

10. Support de stockage non transitoire lisible par ordinateur selon la revendication 9, dans lequel la détection de la troisième région de lésion inclut
la détection d'une région qui correspond à la troisième région fermée dans la première image tomographique comme étant la troisième région de lésion dans la première image tomographique lorsque la troisième région de lésion n'est pas détectée à partir de la première image tomographique sélectionnée.
